# FASCICULE DE BREVET EUROPEEN

(11) **EP 2 742 973 B1**
(45) Date de publication et mention de la délivrance du brevet: **18.02.2015**
(21) Numéro de dépôt: 13185174.3
(22) Date de dépôt: 19.09.2013
(51) Int. Cl.: A61N 1/37, A61B 5/0452

(54) **Dispositif médical implantable actif de type stimulateur cardiaque à détection de stimulation anodique par analyse de vectogramme**
Aktive implantierbare medizinische Vorrichtung vom Typ Herzschrittmacher mit Nachweis der anodischen Stimulation durch Vektogrammanalyse
Active implantable medical device such as a pacemaker with anodal stimulation detection by vector cardiogram analysis

(30) Priorité: 14.12.2012 FR 1262074
(43) Date de publication de la demande: 18.06.2014
(73) Titulaire: Sorin CRM SAS, 92140 Clamart Cedex (FR)
(72) Inventeur: Euzen, Marie-Anen, 91570 Bievres (FR); Vincent, Elodie, 92160 Antony (FR); Graindorge, Laurence, 44470 Thouaré sur Loire (FR)
(74) Mandataire: Dupuis-Latour, Dominique

(56) Documents cités:
- EP-A1- 2 105 843
- EP-A1- 2 324 885
- EP-A1- 2 368 493
- US-A1- 2008 071 318
- US-A1- 2009 030 470
- US-A1- 2010 262 204

## Description

L'invention concerne les "dispositifs médicaux implantables actifs" tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

La stimulation antibradycardique implique la délivrance contrôlée d'impulsions à l'oreillette et/ou au ventricule (dispositifs de type simple ou double chambre). Dans le cas des thérapies de resynchronisation cardiaque CRT, la stimulation doit en outre être appliquée conjointement aux deux ventricules (dispositifs de type multisite).

L'invention vise plus particulièrement la détection du phénomène dit "stimulation anodique", qui se caractérise par une inversion de la cathode et de l'anode lors de la stimulation, avec pour conséquence que la stimulation n'est pas délivrée de la manière désirée et que l'onde de dépolarisation induite par cette stimulation impropre n'est pas celle attendue, avec tous les effets délétères qui peuvent en résulter.

Certaines conditions favorisent l'apparition de ce phénomène, en particulier :
- des amplitudes de stimulation relativement élevées, mais cependant proches du seuil de stimulation ; et
- une configuration de type "vecteur de stimulation intercavité", c'est-à-dire une configuration dans laquelle une stimulation est appliquée dans une cavité donnée (par exemple le ventricule gauche) avec l'anode sur une sonde implantée dans la cavité opposée (par exemple une électrode d'une sonde ventriculaire droite).

Ces conditions se présentent typiquement pendant l'exécution d'un "test de capture", qui est un test consistant à déterminer si une stimulation a été efficace ou non par recherche et analyse de l"'onde évoquée", c'est-à-dire de l'onde de dépolarisation induite par la stimulation d'une cavité. Ce test doit être effectué à intervalles réguliers, et peut également être effectué de façon permanente, cycle-à-cycle. Il a pour but d'ajuster régulièrement l'amplitude et/ou la largeur des impulsions de stimulation, c'est-à-dire l'énergie délivrée au site de stimulation, en fonction de la réponse du myocarde à ces impulsions.

Dans certains cas, la détection du seuil de stimulation doit être opérée de manière indirecte, avec une détection dans une cavité différente de celle qui est stimulée, par exemple par stimulation du ventricule gauche seul et détection de signaux recueillis dans le ventricule droit. Dans une telle situation, les cycles avec stimulation anodique doivent être exclus de l'analyse du signal, car ils ne sont pas représentatifs et ne peuvent donc pas être correctement discriminés en cycles capturants ou non-capturants.

La stimulation anodique produit également des effets délétères chez les patients faisant l'objet d'une thérapie CRT de resynchronisation cardiaque, dans laquelle il est prévu d'appliquer un délai non nul entre les deux stimulations ventriculaires (délai interventriculaire ou DVV), ajusté de manière à optimiser la réponse hémodynamique du patient. Dans ces conditions, une stimulation anodique se caractérisera par exemple par une stimulation du ventricule gauche entrainant une contraction concomitante du ventricule droit, donc une perte du DVV. Ce phénomène n'est pas rare chez les patients faisant l'objet d'une stimulation biventriculaire, et s'il peut être détecté le dispositif pourra prendre des mesures appropriées, par exemple la modification de la configuration de stimulation avec basculement vers un autre vecteur de stimulation avec lequel il n'y aura pas de stimulation anodique (tel qu'un vecteur bipolaire, un vecteur unipolaire, ou un autre vecteur interventriculaire par sélection d'autres électrodes de la sonde).

Diverses techniques ont été proposées pour détecter l'apparition ou la présence d'une stimulation anodique, décrites par exemple dans les US 2009/0030470 A1, US 2010/0262204 A1, US 2008/0071318 A1, US 6 687 545 B1 ou encore WO 2012/071331 A2.

Ces méthodes de détection connues sont en général basées sur l'observation du fait que si une stimulation dans une cavité donnée provoque une dépolarisation immédiate dans la cavité opposée, ceci révèle la présence d'une stimulation anodique, tandis qu'une dépolarisation tardive dans cette cavité opposée indique une absence de stimulation anodique. Avec ces techniques connues, la détection est basée sur l'analyse d'un signal d'électrogramme endocavitaire (signal EGM), ce qui a pour inconvénient que la détection dépend de la qualité de ce même EGM. Il est possible que la qualité de l'EGM soit dégradée sur la voie où il est recueilli, ce qui entraine un risque de sous-évaluation de la stimulation anodique, avec des répercussions possibles sur l'efficacité de la thérapie appliquée au patient.

Le point de départ de l'invention consiste à recueillir et combiner deux EGMs provenant de la même cavité, afin de réduire les risques de diagnostic erroné de présence/absence de stimulation anodique, risques liés à la qualité de l'EGM recueilli. En effet, il est possible que la qualité de l'EGM soit dégradée sur l'une des voies, alors que la qualité reste correcte sur une autre voie EGM de cette même cavité.

Plus précisément, l'idée de base de l'invention consiste à combiner ces deux signaux de manière à permettre une analyse bidimensionnelle de la "boucle cardiaque" ou "vectogramme" (VGM), qui est la représentation dans un espace à deux dimensions de l'un des deux signaux EGM (en ordonnée) par rapport à l'autre (en abscisse), chaque battement ou fraction significative de battement étant alors représenté par son vectogramme dans le plan ainsi défini - et en faisant donc abstraction de la dimension temporelle.

On soulignera que ce "vectogramme" (VGM), qui est obtenu à partir de signaux d'électrogramme (EGM) issus de sondes intracardiaques, ne doit pas être confondu avec le "vectocardiogramme" (VCG) qui est, lui, obtenu à partir de signaux d'électrocardiogramme (ECG) issus d'électrodes externes placées sur le thorax du patient.

Les deux voies EGM différentes peuvent en particulier être celle d'un signal unipolaire (signal lointain ou *far-field* recueilli entre le boitier et une électrode distale ou bien proximale de la sonde), et celle d'un signal bipolaire (signal proche ou *near-field* recueilli entre une électrode distale et une électrode proximale de cette même sonde).

L'analyse d'un VGM a été déjà proposée à d'autres fins, notamment par le EP 2 105 843 A1 (ELA Medical) pour la discrimination entre tachycardies ventriculaires (TV) et supraventriculaires (TSV), par le EP 2 324 885 A1 (Sorin CRM) pour l'invalidation d'un test de capture en cas de fusion, c'est-à-dire de stimulation déclenchée de façon concomitante à une dépolarisation spontanée, ou encore par le EP 2 368 493 A1 (Sorin CRM) pour la discrimination d'artefacts de bruit pour la validation ou l'invalidation de cycles cardiaques à analyser, en particulier pour l'application de thérapies de défibrillation ou de stimulation antitachycardiques (ATP).

Plus précisément, l'invention propose un dispositif comprenant des moyens de détection de stimulation anodique du type général comprenant : des moyens de délivrance d'impulsions électriques de stimulation, appliquées à des électrodes aptes à être implantées dans au moins une cavité cardiaque d'un patient ; des moyens de recueil de signaux EGM d'électrogramme endocavitaire représentatifs de potentiels cardiaques de dépolarisation ; et des moyens de détection de stimulation anodique, aptes à détecter une dépolarisation induite dans une seconde cavité cardiaque par une stimulation délivrée à une première cavité cardiaque, opposée à la première.

De façon caractéristique de l'invention, les moyens de détection de stimulation anodique comprennent : des moyens aptes à délivrer une impulsion de stimulation dans la première cavité au cours d'au moins un cycle cardiaque ; des moyens aptes à recueillir concurremment dans la seconde cavité au moins deux signaux EGM distincts et en dériver au moins deux composantes temporelles distinctes respectives ; des moyens aptes à combiner les deux composantes temporelles en une caractéristique 2D non-temporelle représentative du cycle cardiaque à analyser, à partir des variations de l'une des composantes temporelles en fonction de l'autre ; et des moyens discriminateurs, aptes à déterminer la présence ou l'absence d'une stimulation anodique par analyse de la caractéristique 2D non-temporelle.

Selon diverses caractéristiques subsidiaires avantageuses :
- le dispositif comprenant une sonde ventriculaire apte à être implantée dans la seconde cavité et comportant une électrode distale, une électrode proximale et éventuellement un bobinage de défibrillation, l'une des composantes temporelles est dérivée d'un signal EGM *near-field* bipolaire recueilli entre l'électrode distale et l'électrode proximale de la sonde ventriculaire, et l'autre des composantes temporelles est dérivée d'un signal EGM *far-field* unipolaire recueilli entre d'une part l'électrode distale, l'électrode proximale ou le bobinage de défibrillation (36) de la sonde ventriculaire, et d'autre part un boitier métallique de générateur du dispositif ;
- les moyens aptes à déterminer la caractéristique 2D non-temporelle sont des moyens aptes à déterminer cette caractéristique à partir des variations des composantes temporelles sur une fraction du cycle cardiaque à analyser, dans une fenêtre temporelle d'analyse, ouverte à l'instant de délivrance de l'impulsion de stimulation dans la première cavité ou décalée par rapport à cet instant ;
- le dispositif comprend en outre des moyens pour raccourcir temporairement un délai atrioventriculaire du dispositif pendant l'activation des moyens de détection de stimulation anodique, de telle manière qu'une contraction ventriculaire spontanée survenant après une absence de dépolarisation ventriculaire recueillie se situe hors de la fenêtre temporelle d'analyse ;
- les moyens discriminateurs comprennent : des moyens pour définir un domaine prédéterminé dans un repère correspondant à l'espace des deux composantes temporelles ; et des moyens d'analyse topologique, pour déterminer si la caractéristique 2D non-temporelle est ou non incluse dans le domaine, et décider i) l'absence de stimulation anodique dans le premier cas et ii) la présence d'une stimulation anodique dans le second cas ;
- ce domaine est un domaine rectangulaire, et il est centré sur le point d'origine du repère correspondant à l'espace des deux composantes temporelles ;
- la caractéristique 2D non-temporelle est une caractéristique 2D échantillonnée décrite par une série de points discrets successifs, et les moyens d'analyse topologique sont aptes à analyser la position relative de chaque point par rapport au domaine ;
- les moyens d'analyse topologique sont aptes à décider que la caractéristique 2D n'est pas incluse dans le domaine dès lors qu'au moins un point de la caractéristique 2D échantillonnée se situe hors du domaine, ou bien dès lors qu'au moins deux points de la caractéristique 2D échantillonnée se situent hors du domaine, notamment au moins deux points consécutifs ;
- des caractéristiques 2D non-temporelles sont déterminées pour une pluralité de cycles cardiaques successifs respectifs, et les moyens d'analyse topologique sont aptes à décider la présence de stimulation anodique si au moins l'une des caractéristiques ainsi déterminées est hors du domaine, et l'absence d'une stimulation anodique dans le cas contraire ;
- le dispositif comprend en outre : des moyens de test de capture, aptes à détecter la survenue d'une onde de dépolarisation induite dans la première cavité ou dans une cavité opposée par la stimulation de cette première cavité ; et des moyens aptes à inhiber les moyens de test de capture sur détection d'une stimulation anodique par les moyens discriminateurs.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue générale montrant notamment une sonde bipolaire implantée au fond du ventricule droit du coeur.
La Figure 2 illustre les signes EGM obtenus respectivement sur les voies bipolaire ventriculaire et unipolaire ventriculaire de la sonde de la Figure 1.
La Figure 3 est un exemple de signaux EGM obtenus sur une pluralité de cycles successifs, dont certains présentent un phénomène de stimulation anodique, et d'autres ne le présentent pas.
Les Figures 4a et 4b sont des vues agrandies de deux cycles isolés, respectivement sans et avec stimulation anodique, montrant notamment la fenêtre d'analyse appropriée à la détection de ce phénomène.
La Figure 5 illustre la manière de combiner entre eux les signaux bipolaire et unipolaire de la Figure 2 pour obtenir une caractéristique 2D de type vectogramme (VGM).
Les Figures 6a et 6b illustrent les vectogrammes obtenus, respectivement sans et avec stimulation anodique, et la manière de discriminer entre ces deux situations par analyse topologique du vectogramme.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal.

Comme cela a été indiqué plus haut, la technique de détection de l'invention consiste à analyser l'onde évoquée consécutive à la stimulation d'une cavité à partir de signaux d'électrogramme (EGM) recueillis sur deux voies distinctes et analysés en deux dimensions.

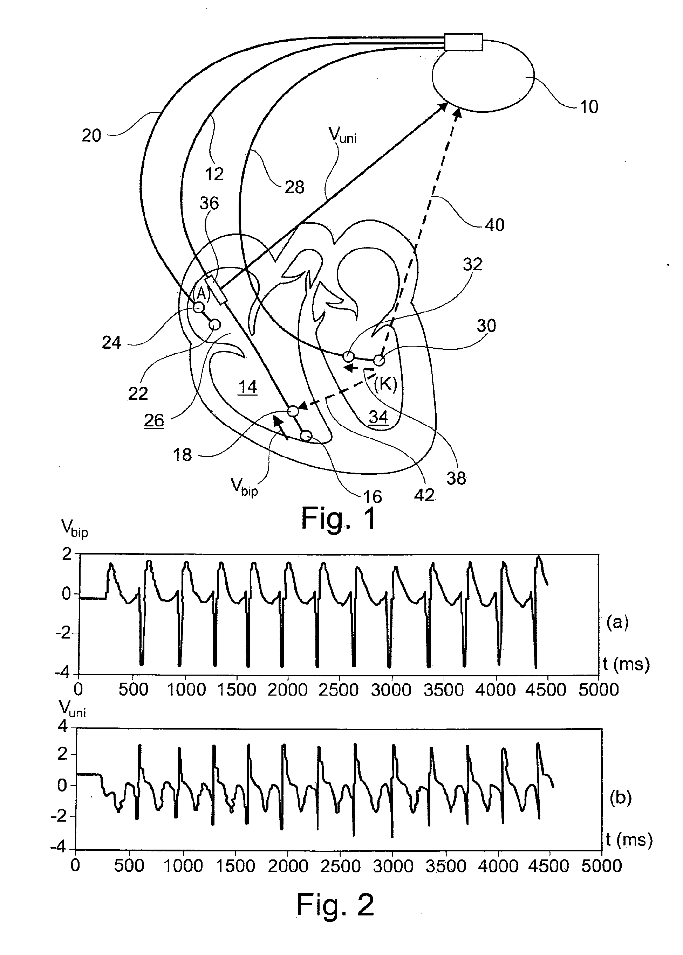

La Figure 1 illustre une configuration de stimulation dans laquelle un générateur d'impulsions 10 est associé à une première sonde 12 implantée dans le ventricule droit 14. La tête de cette sonde comporte deux électrodes, à savoir une électrode distale (*tip*) 16 et une électrode proximale (*ring*) 18.

Une sonde auriculaire 20, pourvue d'électrodes de détection distale 22 et proximale 24 peut être placée dans l'oreillette droite 26 pour la détection des signaux dans cette cavité et l'application éventuelle d'une stimulation auriculaire.

Dans le cas d'une stimulation biventriculaire, destinée notamment à rétablir la synchronisation entre les deux ventricules, le dispositif est pourvu d'une deuxième sonde ventriculaire 28, par exemple une sonde disposée dans le réseau coronaire et comportant une ou plusieurs électrodes 30, 32 disposées au voisinage du ventricule gauche 34. Il est ainsi possible d'assurer la stimulation concomitante, ou avec un léger décalage temporel contrôlé (délai interventriculaire DVV) des deux ventricules droit et gauche pour rétablir la synchronisation entre ces deux cavités et améliorer l'hémodynamique générale du patient.

La sonde ventriculaire droite 12 peut également être pourvue d'un bobinage (*coil*) ventriculaire 36 formant électrode de défibrillation, et permettant aussi de recueillir un signal endocavitaire EGM.

Pour la stimulation du ventricule gauche, il est possible d'utiliser une configuration bipolaire (entre les deux électrodes 30 et 32 de la sonde 28) ou unipolaire (entre l'une des électrodes 30 ou 32 et le boitier (*can*) du générateur 10). Les deux vecteurs de stimulation correspondants sont référencés 38 et 40 sur la Figure 1.

Le phénomène de stimulation anodique peut apparaitre lorsque le retour du courant ne se fait pas sur l'électrode 32 (en stimulation bipolaire) ou sur le boitier du générateur 10 (en stimulation unipolaire), mais sur une électrode située dans le ventricule opposé, par exemple sur l'électrode annulaire 18 de la sonde ventriculaire droite 12 qui forme alors l'anode du vecteur de stimulation 42 (au lieu du vecteur 38 ou 40, selon le cas), la cathode correspondant à l'électrode où est appliquée l'impulsion de stimulation par le générateur, en l'espèce l'électrode distale ventriculaire gauche 30 sur l'exemple de la Figure 1.

Ce phénomène a pour conséquence que l'onde de dépolarisation correspondant à l'impulsion appliquée à gauche va se propager essentiellement dans le ventricule droit, faisant perdre le bénéfice de la stimulation à gauche et donc de la thérapie de resynchronisation.

Dans le cas d'un test de seuil de capture, qui commence avec une énergie de stimulation élevée, le risque d'apparition d'une stimulation anodique est relativement important. Il s'amenuise ensuite au fur et à mesure de la réduction de l'amplitude de stimulation, mais l'algorithme de test aura été leurré en interprétant - à tort - la disparition de la stimulation anodique par une perte de capture, conduisant à une classification erronée des cycles successifs du test entre cycles capturants et non-capturants.

Le but de l'invention est de remédier à cette confusion entre perte de capture et disparition de la stimulation anodique, par une nouvelle technique de détection de l'apparition du phénomène de stimulation anodique permettant de discriminer exactement les cycles ventriculaires présentant un tel phénomène et exclure ceux-ci de l'analyse opérée par l'algorithme de test de capture.

Pour détecter la présence éventuelle d'un tel phénomène, l'invention propose de combiner deux EGMs issus de la même cavité, en l'espèce issus du ventricule droit (c'est-à-dire de la cavité qui n'est pas destinée à être stimulée par l'application d'une impulsion de stimulation sur le ventricule gauche) avec par exemple, comme illustré Figure 1 :
- une composante *Vbip* dérivée d'un signal EGM *near-field* bipolaire recueilli entre l'électrode distale 16 et l'électrode proximale 18 de la sonde ventriculaire droite 12, et
- l'autre composante *Vuni* dérivée d'un signal EGM *far-field* unipolaire recueilli entre le bobinage de défibrillation 36 de la sonde ventriculaire droite 12 et le boitier métallique du générateur 10.

D'autres configurations peuvent être utilisées, à partir de signaux de type *far-field* (par exemple entre l'une des électrodes 16 ou 18 et le boitier 10) et de type *near-field* provenant de la cavité ventriculaire qui ne devrait normalement pas être stimulée (en l'absence de stimulation anodique), à savoir le ventricule droit dans l'exemple illustré.

La Figure 2 illustre un exemple de tracés d'électrogrammes *Vbip* et *Vuni* observés respectivement sur la voie bipolaire ventriculaire (tracé *a*) et sur la voie unipolaire ventriculaire (tracé *b*) de la configuration de la Figure 1.

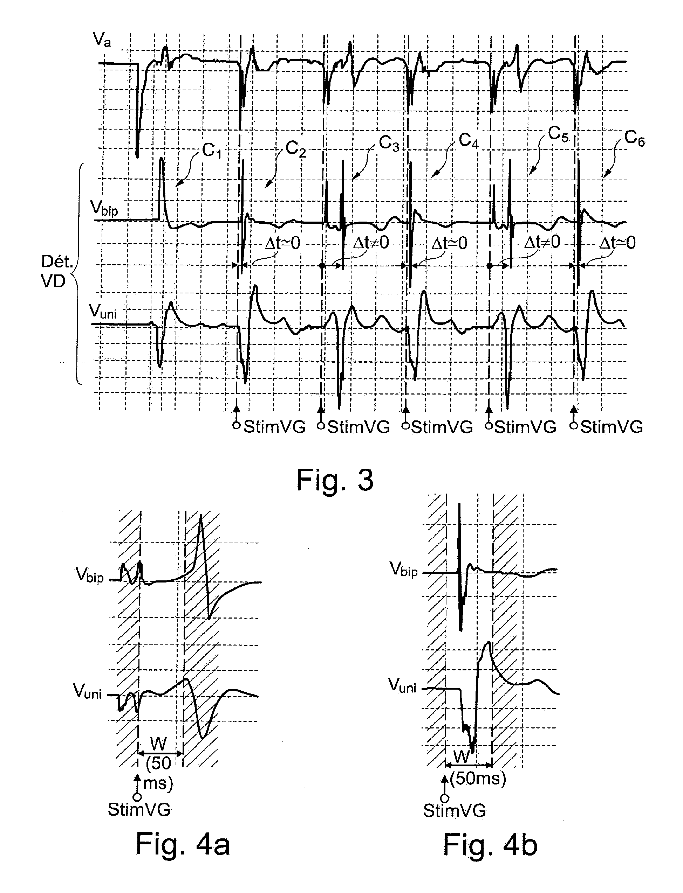

La Figure 3 est un exemple de signaux EGM recueillis, avec les chronogrammes des signaux EGM ventriculaires *Vbip* et *Vuni* ainsi que le signal EGM auriculaire *Va.* De plus, des marqueurs *stim VG* indiquent les instants d'application de la stimulation ventriculaire gauche.

Sur cette figure, six cycles C₁ à C₆ sont visibles, les cycles C₂ à C₆ étant des cycles recueillis côté droit en présence d'une stimulation à gauche (et uniquement à gauche). Ces cycles peuvent être par exemple ceux recueillis lors d'un test de capture à amplitude variable, pour les valeurs les plus élevées de l'amplitude (de l'ordre de 4,5 V), qui comme expliqué plus haut favorisent l'apparition d'un phénomène de stimulation anodique.

Sur les cinq cycles, les cycles C₂, C₄ et C₆ présentent un phénomène de stimulation anodique, avec une dépolarisation détectée sur le ventricule droit pratiquement concomitante à l'application de la stimulation du ventricule gauche (Δt ≈ 0) : en d'autres termes, il y a capture à droite de la stimulation qui a été appliquée à gauche.

En revanche, les cycles C₃ et C₅ ne présentent pas ce phénomène de stimulation anodique : il existe un délai Δt ≠ 0 entre la stimulation du ventricule gauche et l'arrivée de l'onde de dépolarisation, conduite naturellement du ventricule gauche au ventricule droit et détectée sur les EGMs *Vbip* et *Vuni* du ventricule droit.

Il est donc possible d'identifier les cycles dont la dépolarisation provient d'une stimulation anodique en étudiant en détail les composantes du signal pendant ce délai de retard Δt, ce qui peut être notamment réalisé en définissant, comme illustré Figures 4a et 4b, une fenêtre de largeur W, par exemple W = 50 ms, la fenêtre étant comptée à partir de l'instant *stim VG* d'application de l'impulsion de stimulation. Cette fenêtre permet notamment de recueillir des échantillons des signaux *Vbip* et *Vuni* i) qui correspondent à un signal de ligne de base dans le cas d'un cycle avec capture ou d'un cycle sans capture et ii) qui correspondent à une dépolarisation dans le cas d'une stimulation anodique.

La longueur W de la fenêtre peut être différente de la valeur indiquée dans cet exemple, et le début de la fenêtre peut être éventuellement décalé dans le temps par rapport à l'instant de la stimulation. La largeur W et l'instant de début de la fenêtre peuvent être également des valeurs para-métrables, pour permettre leur adaptation à chaque patient.

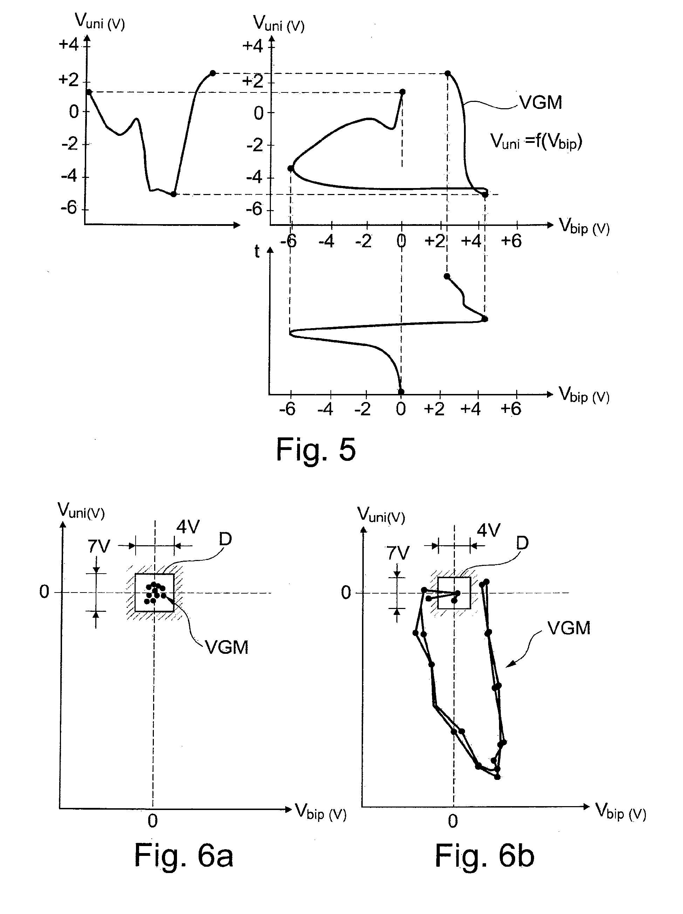

La Figure 5 montre la manière de combiner entre elles les deux composantes temporelles *Vbip* et *Vuni* pour obtenir une caractéristique 2D de type vectogramme (VGM) permettant de discriminer de manière fiable entre cycles avec et sans stimulation anodique.

Concrètement, les signaux EGM *Vuni* (*t*) et *Vbip*(*t*) recueillis sont échantillonnés et les échantillons successifs des deux composantes ainsi recueillis sont mémorisés puis combinés entre eux pour éliminer la variable temporelle *t* et produire une courbe paramétrique (la caractéristique VGM) du type *Vuni* = f(*Vbip*).

Cette courbe *Vuni* = f(*Vbip*) est une courbe paramétrique sans dimension temporelle, tracée à partir des variations de l'une des composantes temporelles (*Vuni*) en fonction de l'autre (*Vbip*). Elle constitue un vectocardiogramme (VGM) représentatif du cycle cardiaque à analyser (ou d'une fraction de ce cycle), et sera désignée par la suite "caractéristique 2D non-temporelle". Elle présente graphiquement la forme d'une boucle, le temps n'apparaissant plus que dans la manière dont la boucle est parcourue sur la durée du cycle ou de la fraction de cycle.

On notera incidemment que l'analyse 'bidimensionnelle" ou "en deux dimensions" (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

Par ailleurs, il n'est pas indispensable d'analyser la totalité du cycle, l'analyse d'une fraction significative de ce cycle (celle correspondant à la fenêtre d'analyse de largeur W) étant en général suffisante. Dans ce cas, la caractéristique curviligne représentative du VGM n'est pas une boucle fermée, dans la mesure où elle ne correspond qu'à une partie du cycle cardiaque complet, à savoir le complexe QRS isolé à l'intérieur de la fenêtre d'analyse.

De façon caractéristique de l'invention, la présence ou l'absence d'une stimulation anodique est détectée par une analyse de cette caractéristique VGM.

Une première technique d'analyse consiste à opérer une analyse morphologique de la caractéristique VGM, considérée en tant que telle sur le cycle à analyser. Des paramètres descripteurs du vectogramme sont ainsi calculés et analysés, qui peuvent être notamment les angles des vecteurs tangents respectifs considérés en divers points de la caractéristique VGM, ou encore la courbure de cette caractéristique VGM, ou bien une combinaison de plusieurs paramètres, par exemple une combinaison de la norme et de l'angle des vecteurs tangents.

Diverses variantes de ces techniques d'analyse morphologique, utilisées à d'autres fins, sont décrites notamment dans le EP 2 368 493 A1 précité, auquel on pourra se reporter pour de plus amples détails.

Toutefois, une technique d'analyse particulièrement avantageuse consiste à examiner la caractéristique VGM par rapport à un domaine prédéterminé défini dans le repère où elle est tracée, par exemple par rapport à un domaine rectangulaire de dimensions et de position prédéfinies, et de procéder à une analyse topologique de la distribution des points de la caractéristique VGM par rapport à ce domaine : la position de ces points à l'intérieur ou à l'extérieur du domaine prédéterminé constituera le critère utilisé pour décider de la présence ou de l'absence d'une stimulation anodique.

Plus précisément, comme illustré sur les Figures 6a et 6b, on constate qu'en cas de stimulation anodique, la caractéristique VGM, déterminée par les points successifs d'échantillonnage des signaux *Vbip* et *Vuni,* est simplement constituée d'un nuage de points centrés approximativement autour du point de coordonnées {0,0}, comme illustré Figure 6a. En revanche, en cas de stimulation anodique la caractéristique VGM a une allure de courbe, comme illustré Figure 6b (une courbe ouverte si l'échantillonnage se fait sur une fraction seulement du cycle cardiaque).

L'idée de base de l'invention consiste à définir un domaine D dans le repère {*Vuni*, *Vbip*} où est tracée la caractéristique VGM, à superposer ce domaine à la caractéristique VGM, à évaluer si cette caractéristique VGM est ou non contenue dans le domaine, et à décider selon le cas l'absence ou la présence d'une stimulation anodique.

Le domaine D peut être avantageusement, comme illustré Figures 6a et 6b, un domaine rectangulaire centré sur le point de coordonnées {0,0}, de côtés 4 V (pour *Vbip*) x 7 V (pour *Vuni*).

Le critère de décision sera par exemple le suivant :
- si tous les points de la caractéristique VGM sont à l'intérieur de ce rectangle, alors il n'y a pas stimulation anodique ;
- si au moins un point de cette caractéristique se situe hors du rectangle, alors il y a stimulation anodique.

Des critères différents peuvent être utilisés, par exemple imposant la présence de deux points au moins de la caractéristique VGM hors du rectangle pour décider la présence d'une stimulation anodique, voire même deux points consécutifs hors du rectangle.

On dispose ainsi d'un moyen très simple et très efficace de discriminer les cycles avec stimulation anodique, sans mise en oeuvre de techniques complexes d'analyse morphologique de la caractéristique VGM, ni comparaison de cette caractéristique à des modèles de référence préalablement acquis et nécessitant une mise à jour régulière.

En particulier, cette technique de détection est tout à fait compatible avec ce dont il est possible de disposer dans un implant conventionnel, dont le processeur et les mémoires sont sollicités par la mise en oeuvre de très nombreuses fonctions de détection et de calcul, et qu'il importe de ne pas surcharger.

Bien sûr, pour mettre en oeuvre cette technique de discrimination des cycles avec stimulation anodique, il est nécessaire de se mettre dans des conditions telles qu'un cycle ventriculaire spontané présent malgré l'absence d'une capture n'apparaisse pas dans la fenêtre d'analyse des signaux *Vbip* et *Vuni* utilisés pour tracer la caractéristique VGM. Pour ce faire, il convient de programmer temporairement le dispositif avec un délai atrioventriculaire DAV très court, pendant la durée du test de capture mettant en oeuvre la technique de discrimination des cycles en stimulation anodique selon l'invention.

## Revendications

1. Un dispositif médical actif, comprenant :
- des moyens de délivrance d'impulsions électriques de stimulation, appliquées à des électrodes aptes à être implantées dans au moins une cavité cardiaque d'un patient ;
- des moyens de recueil de signaux EGM d'électrogramme endocavitaire représentatifs de potentiels cardiaques de dépolarisation ; et
- des moyens de détection de stimulation anodique, aptes à détecter une dépolarisation induite dans une seconde cavité cardiaque (14) par une stimulation délivrée à une première cavité cardiaque (34), opposée à la première,
**caractérisé en ce que** les moyens de détection de stimulation anodique comprennent :
- des moyens (10, 30, 32) aptes à délivrer une impulsion de stimulation dans la première cavité (14) au cours d'au moins un cycle cardiaque ;
- des moyens (10, 16, 18, 36) aptes à recueillir concurremment dans la seconde cavité (34) au moins deux signaux EGM distincts et en dériver au moins deux composantes temporelles distinctes (*Vbip*, *Vuni*) respectives ;
- des moyens aptes à combiner les deux composantes temporelles en une caractéristique 2D non-temporelle (VGM) représentative du cycle cardiaque à analyser, à partir des variations de l'une des composantes temporelles (*Vuni*) en fonction de l'autre (*Vbip*) ; et
- des moyens discriminateurs, aptes à déterminer la présence ou l'absence d'une stimulation anodique par analyse de la caractéristique 2D non-temporelle.

2. Le dispositif de la revendication 1, dans lequel :
- le dispositif comprend une sonde ventriculaire (20) apte à être implantée dans la seconde cavité, cette sonde ventriculaire comprenant une électrode distale (16), une électrode proximale (18) et éventuellement un bobinage de défibrillation (36) ;
- l'une des composantes temporelles (*Vbip*) est dérivée d'un signal EGM *near-field* bipolaire recueilli entre l'électrode distale (16) et l'électrode proximale (18) de la sonde ventriculaire ; et
- l'autre des composantes temporelles (*Vuni*) est dérivée d'un signal EGM *far-field* unipolaire recueilli entre d'une part l'électrode distale (16), l'électrode proximale (16) ou le bobinage de défibrillation (36) de la sonde ventriculaire, et d'autre part un boitier métallique de générateur (10) du dispositif.

3. Le dispositif de la revendication 1, dans lequel les moyens aptes à déterminer la caractéristique 2D non-temporelle sont des moyens aptes à déterminer cette caractéristique à partir des variations des composantes temporelles sur une fraction du cycle cardiaque à analyser, dans une fenêtre temporelle d'analyse, ouverte à l'instant (*StimVG*) de délivrance de l'impulsion de stimulation dans la première cavité ou décalée par rapport à cet instant.

4. Le dispositif de la revendication 3, dans lequel le dispositif comprend en outre des moyens pour raccourcir temporairement un délai atrioventriculaire du dispositif pendant l'activation des moyens de détection de stimulation anodique, de telle manière qu'une contraction ventriculaire spontanée survenant après une absence de dépolarisation ventriculaire recueillie se situe hors de la fenêtre temporelle d'analyse.

5. Le dispositif de la revendication 1, dans lequel les moyens discriminateurs comprennent :
- des moyens pour définir un domaine prédéterminé (D) dans un repère correspondant à l'espace des deux composantes temporelles ; et
- des moyens d'analyse topologique, pour déterminer si la caractéristique 2D non-temporelle (VGM) est ou non incluse dans le domaine, et décider i) l'absence de stimulation anodique dans le premier cas et ii) la présence d'une stimulation anodique dans le second cas.

6. Le dispositif de la revendication 5, dans lequel le domaine est un domaine rectangulaire.

7. Le dispositif de la revendication 5, dans lequel le domaine est centré sur le point d'origine du repère correspondant à l'espace des deux composantes temporelles.

8. Le dispositif de la revendication 5, dans lequel la caractéristique 2D non-temporelle est une caractéristique 2D échantillonnée décrite par une série de points discrets successifs, et dans lequel les moyens d'analyse topologique comprennent des moyens d'analyse de la position relative de chaque point par rapport au domaine.

9. Le dispositif de la revendication 5, dans lequel les moyens d'analyse topologique comprennent des moyens pour vérifier si au moins un point de la caractéristique 2D échantillonnée se situe hors du domaine et décider alors que la caractéristique 2D n'est pas incluse dans le domaine.

10. Le dispositif de la revendication 5, dans lequel les moyens d'analyse topologique comprennent des moyens pour vérifier si au moins deux points de la caractéristique 2D échantillonnée se situent hors du domaine et décider alors que la caractéristique 2D n'est pas incluse dans le domaine.

11. Le dispositif de la revendication 5, dans lequel les moyens d'analyse topologique comprennent des moyens pour vérifier si au moins deux points consécutifs de la caractéristique 2D échantillonnée se situent hors du domaine et décider alors que la caractéristique 2D n'est pas incluse dans le domaine.

12. Le dispositif de la revendication 1, dans lequel :
- des caractéristiques 2D non-temporelles sont déterminées pour une pluralité de cycles cardiaques successifs respectifs ; et
- les moyens d'analyse topologique comprennent des moyens pour vérifier si au moins l'une des caractéristiques ainsi déterminées est hors du domaine et décider alors la présence de stimulation anodique, et l'absence d'une stimulation anodique dans le cas contraire.

13. Le dispositif de la revendication 1, comprenant en outre :
- des moyens de test de capture, aptes à détecter la survenue d'une onde de dépolarisation induite dans la première cavité ou dans une cavité opposée par la stimulation de cette première cavité ; et
- des moyens aptes à inhiber les moyens de test de capture sur détection d'une stimulation anodique par les moyens discriminateurs.

## Patentansprüche

1. Aktive medizinische Vorrichtung, die enthält:
- Einrichtungen zum Liefern elektrischer Stimulationsimpulse, die an Elektroden angelegt werden, die in mindestens eine Herzkammer eines Patienten eingesetzt werden können;
- Einrichtungen zum Auffangen von EGM-Signalen eines endokavitären Elektrogramms, die für Herz-Depolarisationspotentiale repräsentativ sind; und
- Einrichtungen zur Erfassung einer Anodenstimulation, die eine Depolarisation erfassen können, die durch eine an eine erste Herzkammer (34) gelieferte Stimulation in eine zweite Herzkammer (14) entgegengesetzt zur ersten induziert wird,
**dadurch gekennzeichnet, dass** die Einrichtungen zur Erfassung einer Anodenstimulation enthalten:
- Einrichtungen (10, 30, 32), die während mindestens eines Herzzyklus einen Stimulationsimpuls in die erste Kammer (14) liefern können;
- Einrichtungen (10, 16, 18, 36), die gleichzeitig in der zweiten Kammer (34) mindestens zwei unterschiedliche EGM-Signale auffangen und daraus mindestens zwei unterschiedliche Zeitkomponenten (Vbip, Vuni) ableiten können;
- Einrichtungen, die die zwei Zeitkomponenten in einem nicht-zeitlichen 2D-Merkmal (VGM) kombinieren können, das für den zu analysierenden Herzzyklus repräsentativ ist, ausgehend von Schwankungen einer der Zeitkomponenten (Vuni) abhängig von der anderen (Vbip); und
- Diskriminatoreinrichtungen, die das Vorhandensein oder die Abwesenheit einer Anodenstimulation durch Analyse des nicht-zeitlichen 2D-Merkmals ermitteln können.

2. Vorrichtung nach Anspruch 1 , wobei:
- die Vorrichtung eine ventrikuläre Sonde (20) enthält, die in die zweite Kammer eingesetzt werden kann, wobei diese ventrikuläre Sonde eine distale Elektrode (16), eine proximale Elektrode (18) und ggf. eine Defibrillierwicklung (36) enthält;
- eine der Zeitkomponenten (Vbip) von einem bipolaren Near-Field-EGM-Signal abgeleitet wird, das zwischen der distalen Elektrode (16) und der proximalen Elektrode (18) der ventrikulären Sonde aufgefangen wird; und
- die andere der Zeitkomponenten (Vuni) von einem unipolaren Far-Field-EGM-Signal abgeleitet wird, das zwischen einerseits der distalen Elektrode (16), der proximalen Elektrode (16) oder der Defibrillierwicklung (36) der ventrikulären Sonde und andererseits einem metallischen Generatorgehäuse (10) der Vorrichtung aufgefangen wird.

3. Vorrichtung nach Anspruch 1, wobei die Einrichtungen, die das nicht-zeitliche 2D-Merkmal ermitteln können, Einrichtungen sind, die dieses Merkmal ausgehend von den Schwankungen der Zeitkomponenten über einen Bruchteil des zu analysierenden Herzzyklus in einem Analysezeitfenster ermitteln können, das zum Zeitpunkt (StimVG) der Lieferung des Stimulationsimpulses in die erste Kammer oder bezüglich dieses Zeitpunkts versetzt offen ist.

4. Vorrichtung nach Anspruch 3, wobei die Vorrichtung außerdem Einrichtungen enthält, um vorübergehend eine atrioventrikuläre Verzögerung der Vorrichtung während der Aktivierung der Einrichtungen zur Erfassung einer Anodenstimulation zu verkürzen, damit eine nach einer Abwesenheit einer aufgefangenen ventrikulären Depolarisation auftretende spontane ventrikuläre Kontraktion sich außerhalb des Analysezeitfensters befindet.

5. Vorrichtung nach Anspruch 1, wobei die Diskriminatoreinrichtungen enthalten:
- Einrichtungen, um einen vorbestimmten Bereich (D) in einem Bezugssystem zu definieren, der dem Raum der zwei Zeitkomponenten entspricht; und
- Einrichtungen zur topologischen Analyse, um zu ermitteln, ob das nicht-zeitliche 2D-Merkmal (VGM) in dem Bereich enthalten ist oder nicht, und um i) die Abwesenheit einer Anodenstimulation im ersten Fall und ii) das Vorhandensein einer Anodenstimulation im zweiten Fall zu bestimmen.

6. Vorrichtung nach Anspruch 5, wobei der Bereich ein Rechteckbereich ist.

7. Vorrichtung nach Anspruch 5, wobei der Bereich auf den Ursprungspunkt des Bezugssystems zentriert ist, der dem Raum der zwei Zeitkomponenten entspricht.

8. Vorrichtung nach Anspruch 5, wobei das nicht-zeitliche 2D-Merkmal ein abgetastetes 2D-Merkmal ist, das durch eine Reihe von aufeinanderfolgenden diskreten Punkten beschrieben wird, und wobei die Einrichtungen zur topologischen Analyse Einrichtungen zur Analyse der relativen Stellung jedes Punkts bezüglich des Bereichs enthalten.

9. Vorrichtung nach Anspruch 5, wobei die Einrichtungen zur topologischen Analyse Einrichtungen enthalten, um zu überprüfen, ob mindestens ein Punkt des abgetasteten 2D-Merkmals sich außerhalb des Bereichs befindet, und dann zu bestimmen, dass das 2D-Merkmal nicht im Bereich enthalten ist.

10. Vorrichtung nach Anspruch 5, wobei die Einrichtungen zur topologischen Analyse Einrichtungen enthalten, um zu überprüfen, ob mindestens zwei Punkte des abgetasteten 2D-Merkmals sich außerhalb des Bereichs befinden, und dann zu bestimmen, dass das 2D-Merkmal nicht im Bereich enthalten ist.

11. Vorrichtung nach Anspruch 5, wobei die Einrichtungen zur topologischen Analyse Einrichtungen enthalten, um zu überprüfen, ob mindestens zwei aufeinanderfolgende Punkte des abgetasteten 2D-Merkmals sich außerhalb des Bereichs befinden, und dann zu bestimmen, dass das 2D-Merkmal nicht im Bereich enthalten ist.

12. Vorrichtung nach Anspruch 1, wobei:
- nicht-zeitliche 2D-Merkmale für eine Vielzahl von jeweiligen aufeinanderfolgenden Herzzyklen ermittelt werden; und
- die Einrichtungen zur topologischen Analyse Einrichtungen enthalten, um zu überprüfen, ob mindestens eines der so ermittelten Merkmale sich außerhalb des Bereichs befindet, und dann das Vorhandensein einer Anodenstimulation und im gegenteiligen Fall die Abwesenheit einer Anodenstimulation zu bestimmen.

13. Vorrichtung nach Anspruch 1, die außerdem enthält:
- Einfangtest-Einrichtungen, die das Auftreten einer Depolarisationswelle erfassen können, die in die erste Kammer oder in eine entgegengesetzte Kammer durch die Stimulation dieser ersten Kammer induziert wird; und
- Einrichtungen, die bei Erfassung einer Anodenstimulation durch die Diskriminatoreinrichtungen die Einfangtest-Einrichtungen sperren können.

## Claims

1. An active medical device comprising:
- means for delivering electric stimulation pulses, applied to electrodes adapted to be implanted in at least one heart cavity of a patient;
- means for collecting endocavitary electrogram EGM signals representative of depolarisation cardiac potentials; and
- means for detecting an anodic stimulation, adapted to detect a depolarisation induced in a second heart cavity (14) by a stimulation delivered in a first heart cavity (34), opposite to the first one,
**characterized in that** the anodic stimulation detection means comprise:
- means (10, 30, 32) adapted to deliver a stimulation pulse in the first cavity (14) during at least one cardiac cycle;
- means (10, 16, 18, 36) adapted to concurrently collect in the second cavity (34) at least two distinct EGM signals and to derive therefrom at least two respective distinct time components (*V_{bip}*, V*ᵤₙᵢ*) ;
- means adapted to combine the two time components into one non-time 2D characteristic (VGM) representative of the cardiac cycle to be analysed, based on the variations of one of the time components (V*ᵤₙᵢ*) as a function of the other (V*_{bip}*); and
- discriminating means, adapted to determine the presence or absence of an anodic stimulation by analysis of the non-time 2D characteristic.

2. The device of claim 1, wherein:
- the device comprises a ventricular lead (20) adapted to be implanted into the second cavity, this ventricular lead comprising a distal electrode (16), a proximal electrode (18) and possibly a defibrillation winding (36);
- one of the time components (*V_{bip}*) is derived from a near-field bipolar EGM signal collected between the distal electrode (16) and the proximal electrode (18) of the ventricular probe; and
- the other of the time components (V*ᵤₙᵢ*) is derived from a far-field unipolar EGM signal collected between, on the one hand, the distal electrode (16), the proximal electrode (18) or the defibrillation winding (36) of the ventricular lead, and on the other hand, a metal case of a generator (10) of the device.

3. The device of claim 1, wherein the means adapted to determine the non-time 2D characteristic are means adapted to determine this characteristic based on the variations of the time components over a fraction of the cardiac cycle to be analysed, within a time window of analysis, open at the instant (*StimVG*) of delivery of the stimulation pulse in the first cavity or offset relative to this instant.

4. The device of claim 3, wherein the device further comprises means for temporarily shortening an atrioventricular delay of the device during the activation of the anodic stimulation detection means, in such a manner that a spontaneous ventricular contraction occurring after an absence of collected ventricular depolarisation is located out of the time window of analysis.

5. The device of claim 1, wherein the discriminating means comprise:
- means for defining a predetermined domain (D) in a reference system corresponding to the space of the two time components; and
- topological analysis means, for determining whether the non-time 2D characteristic (VGM) is included in the domain, and deciding i) the absence of an anodic stimulation in the first case and ii) the presence of an anodic stimulation in the second case.

6. The device of claim 5, wherein the domain is a rectangular domain.

7. The device of claim 5, wherein the domain is centred on the origin point of the reference system corresponding to the space of the two time components.

8. The device of claim 5, wherein the non-time 2D characteristic is a sampled 2D characteristic described by a series of successive discrete points, and in which the topological analysis means comprise means for analysing the relative position of each point with respect to the domain.

9. The device of claim 5, wherein the topological analysis means comprise means for verifying if at least one point of the sampled 2D characteristic is located out of the domain and then deciding that the 2D characteristic is not included in the domain.

10. The device of claim 5, wherein the topological analysis means comprise means for verifying if at least two points of the sampled 2D characteristic are located out of the domain and then deciding that the 2D characteristic is not included in the domain.

11. The device of claim 5, wherein the topological analysis means comprise means for verifying if at least two consecutive points of the sampled 2D characteristic are located out of the domain and then deciding that the 2D characteristic is not included in the domain.

12. The device of claim 1, wherein:
- non-time 2D characteristics are determined for a plurality of respective successive cardiac cycles; and
- the topological analysis means comprise means for verifying if at least one of the thus-determined characteristic is out of the domain and then deciding the presence of an anodic stimulation, and the absence of an anodic stimulation in the opposite case.

13. The device of claim 1, further comprising:
- capture test means, adapted to detect the occurrence of a depolarisation wave induced in the first cavity or in an opposite cavity by the stimulation of this first cavity; and
- means adapted to inhibit the capture test means upon detection of an anodic stimulation by the discriminating means.
